# EUROPEAN PATENT SPECIFICATION

(11) **EP 1 352 611 B1**
(45) Date of publication and mention of the grant of the patent: **19.11.2008**
(21) Application number: 03252077.7
(22) Date of filing: 01.04.2003
(51) Int. Cl.: A61B 5/00, A61J 1/00, G01N 33/487

(54) **Test strip vial**
Teststreifen Behältnis
Fiole pour bande d' essai

(30) Priority: 02.04.2002 GB 0207609
(43) Date of publication of application: 15.10.2003
(73) Proprietor: Lifescan Scotland Ltd, Inverness-shire IV2 3ED (GB)
(72) Inventor: Griffith, Alun Wyn, Inverness, IV2 3XQ (GB); Moerman, Piet, 9831 St. Marens-Latem (BE); Francovich, Walter, Pierrefonds, Quebec H9J 3S8 (CA); Mainville, Patrick, Montreal, Quebec H2C 2J9 (CA)
(74) Representative: Mercer, Christopher Paul

(56) References cited:
- EP-A- 1 147 739
- WO-A-02/055008

## Description

### Field of the Invention

The present invention relates to a vial for holding and dispensing test strips for a diagnostic apparatus. The test strips may be used for testing analytes in bodily fluids, for example analytes such as, but not limited to, glucose. More particularly, the present invention relates to a vial for storing and dispensing test strips for a glucose meter. The present invention also relates to a cassette for use in such a vial.

### Background of the Invention

Glucose monitoring is a fact of everyday life for diabetic individuals, and the accuracy of such monitoring can literally mean the difference between life and death. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

The process of monitoring blood glucose levels requires several steps and several different accessories, including a lancing device, a lancet, a supply of disposable electrochemical or photometric test strips and a glucose meter.

Many of the glucose meter designs currently available make use of a disposable test strip having an electrode system, which, in combination with the meter, electrochemically measures the amount of glucose in a blood sample. To use these meters, the user first pricks a finger or other body part using a lancing device to produce a small sample of blood or interstitial fluid. Then, the user opens a vial of test strips, removes a test strip and inserts the test strip into the meter. The user then re-closes the vial and checks for the correct calibration code on the meter. The user then transfers the sample to the test strip. Generally, a user is required to transfer a specific volume of sample to a specific location on the small test strip, a difficult task for many users. The electrode system on the test strip generates a signal indicative of an amount of glucose in the blood sample, and transmits this signal to the glucose meter, which displays and stores the reading.

Current test strips and vials for storing test strips present significant limitations. Current test strips are generally about two centimetres in length. This length is considered to be the minimum practical length to allow a user to easily handle the test strips. It is generally desirable to minimise the required length of the test strips, thereby minimising the resources required to produce a test strip and consequently increasing strip production while reducing costs. However, test strips that are shorter than the conventional two centimetres are generally harder to handle, increasing the difficulty of placing and removing the test strip from the meter without dropping the test strip or contaminating the user with blood. Test strip limitations are exacerbated by the fact that diabetics often have poor eyesight and generally already have difficulty handling test strips.

Furthermore, current test strips are usually stored loosely in a vial, which results in an inefficient utilisation of space. A user must open the vial, reach into the vial and grab and remove one of the test strips from the vial interior. The user must then remember to re-seal the vial afterwards or the test strips will deteriorate due to humidity exposure and not be useable. The user must then manually handle the test strip, insert the test strip into the glucose meter, lance the skin, obtain a drop of blood and transfer the blood sample onto the test strip. Finally, after analysis, the user is required to remove the test strip.

WO-A-020 55 008 describes a cassette with test strips.

A pusher bar permits the strips to move out of the cassette. EP 1 147 739 describes also a receptacle with test strips.

It is an aim of the present invention to provide, at least in part, a solution to the problem of handling small test strips.

### Summary of the Invention

In view of the foregoing and in accordance with the present invention, there is provided, in a first aspect, a cassette for holding and dispensing test strips for analysing a sample of bodily fluid, the cassette having a dispensing opening and being configured to hold a supply of test strips, the cassette further including:
biasing means for biasing test strips held in the cassette towards the dispensing opening;
retaining means for holding the test strips in the cassette against the bias of the biasing means; and
means for moving test strips, one at a time, out of the cassette through the dispensing opening, wherein the retaining means is an enclosing surface at the end of the cassette having the dispensing opening therein, and wherein the retaining means includes an longitudinal slot extending along an outer side of the enclosing surface.

The cassette of the present invention is able to store a number of test strips in a compact manner and can be operated to dispense even small strips, one at a time, as required by a user.

Preferably, the biasing means is attached to the cassette.

Preferably, the biasing means is a helical spring.

Preferably, the biasing means is connected to a strip retainer for contacting the supply of test strips.

Preferably, the retaining means is an enclosing surface at the end of the cassette having the dispensing opening therein.

Preferably, the enclosing surface includes a channel in communication with the dispensing opening and configured to hold a single test strip from the supply of test strips.

Preferably, the means for moving is a slider and the enclosing surface includes an elongated slot for holding the slider, such that the slider is in communication with the channel and movement of the slider towards the dispensing opening dispenses a single test strip held in the channel through the dispensing opening.

The use of a slider is advantageous because it is simple, yet reliably delivers only one strip at a time.

Preferably, the slider is biased to return the slider to its starting position after dispensing a test strip through the dispensing opening.

Preferably, the cassette further comprises a window formed in the enclosing surface to allow a user to view a number on a surface of a test strip.

In one embodiment of the present invention, the cassette comprises desiccant material disposed in the cassette for preserving the test strips from humidity.

In another embodiment of the present invention, the cassette is formed of desiccant material.

Preferably, the test strips have a length of one centimetre or less.

In yet another embodiment of the present invention, the cassette is disposable.

Preferably, the bodily fluid is blood and the test strips are used for measurement of a glucose level in the blood.

According to another aspect of the present invention, there is provided a vial comprising the cassette as described above and an outer housing containing the cassette.

In one embodiment of the present invention, the biasing means is attached to the outer housing and communicates with test strips in the cassette through a slot in the cassette.

Preferably, the outer housing is formed of desiccant material.

Preferably, the outer housing has an oval-shaped cross-section.

Preferably, the outer housing includes a depression to facilitate holding of the vial by a user.

Preferably, the cassette includes a protruding portion that protrudes from the outer housing when the cassette is inserted into the outer housing and a hermetic seal is disposed on the protruding portion.

Preferably, the outer housing comprises a lid.

Preferably, the outer housing includes a recessed portion adjacent to an upper rim and the lid includes a protruding portion corresponding to the recessed portion to facilitate opening or removal of the lid.

In one embodiment of the present invention, the vial further comprises data storage means carried by the vial for storing information. This can eliminate the need for a user to enter data, related to the test strips, into a meter manually.

Preferably, the data storage means is an electronic chip.

Preferably, the electronic chip is an EPROM or an EEPROM.

In one embodiment of the present invention, the information stored by the data storage means relates to information about a supply of test strips stored in the vial.

In another embodiment of the present invention, the information comprises at least one of a calibration code for the supply of test strips, an expiration date of the supply of test strips, the batch code of the supply of test strips, the lot number of the supply of test strips and the number of strips in the supply of test strips.

In yet another embodiment of the present invention, the data storage means is adapted to store analyte values obtained by a testing device.

In yet another embodiment of the present invention, the data storage means contains software which can be transferred to a testing device.

Preferably, the software includes instructions for operating the testing device to accommodate a set of operating parameters for a supply of test strips stored in the vial.

In one embodiment of the present invention, the vial further comprises an adapter configured to connect to the contacts of a testing device. This enables the vial to be used more easily with the testing device.

According to yet another aspect of the present invention, there is provided a vial for storing and dispensing test strips for analysing a sample of bodily fluid comprising data storage means affixed to the housing for storing information related to the test strips.

Preferably, the data storage means is an electronic chip.

Preferably, the electronic chip is an EPROM or an EEPROM.

In one embodiment of the present invention, the data storage means is adapted to store bodily fluid readings obtained by a testing device that performs an analysis using the test strips stored in the vial.

In another embodiment of the present invention, the data storage means stores instructions for modifying the operating parameters of the testing device according to the type of test strips stored in the vial.

In yet another embodiment of the present invention, the data storage means stores advertising information about the strips for display on a testing device.

In yet another embodiment of the present invention, the data storage means stores personal information for a user of the vial.

According to yet another aspect of the present invention, there is provided a method of dispensing a test strip for measuring an analyte level in a sample of bodily fluid comprising:
dispensing a test strip from a cassette containing a supply of test strips using a dispensing mechanism,
wherein after dispensing a test strip, biasing means applied to the supply of test strips in the cassette causes a further test strip to be made available for dispensing.

Preferably, the cassette is inserted into an outer housing before dispensing a test strip.

In one embodiment of the present invention, the method further comprises the step of inserting the test strip into a testing device.

Preferably, the test strip is used to measure a glucose level in blood and the testing device is a glucose meter.

In another embodiment of the present invention, the method further comprises the step of disposing of the cassette when the supply of test strips is exhausted.

Accordingly, the present invention provides a test strip vial for storing and dispensing test strips for use with a diagnostic test device such as, but not limited to, a glucose meter. The test strip vial may comprise a hermetically sealed cassette configured to store a stack of test strips and dispense the test strips from the cassette one at a time. The test strip vial may provide simplified loading of a test strip into a glucose meter for measuring the glucose level of a sample of blood. The test strip vial may comprise an outer housing comprised of a base container and a partially attached lid for closing the base container. The vial further includes a cassette inserted in the base container for holding a supply of test strips and dispensing the test strips from the vial on an individual basis using a dispensing mechanism. Biasing means is also provided for biasing the stack of test strips towards the dispensing mechanism to facilitate one-by-one dispensing. The test strip vial may further include an information chip for providing read and write storage capability to the vial. This information may be read by the meter through an alignment feature with an electric contact on both.

### Brief Description of the Drawings

A specific embodiment is now described, by way of example only and with reference to the accompanying drawings, in which:
Figure 1 illustrates a conventional test strip vial for storing a supply of test strips;
Figure 2 is an exploded view of a test strip vial according to an illustrative embodiment of the present invention;
Figures 3a and 3b illustrate the test strip vial of an illustrative embodiment in an assembled, open position;
Figure 4a is a cross-sectional view along the length of a fully loaded and assembled test strip vial of an illustrative embodiment of the present invention;
Figure 4b is a cross-sectional view along the length of a nearly empty and assembled test strip vial of an illustrative embodiment of the present invention;
Figure 4c is a detailed side view of the strip retainer of Figures 4a and 4b;
Figure 5a is a top view of the test strip vial of an illustrative embodiment of the present invention;
Figure 5b is a frontal view of the test strip vial of an illustrative embodiment of the present invention;
Figure 5c is a side view of the test strip vial of an illustrative embodiment of the present invention;
Figure 5d is a back view of the test strip vial of an illustrative embodiment of the present invention;
Figures 6a - 6d illustrate the steps involved in assembling a test strip vial of an illustrative embodiment of the present invention;
Figure 7 illustrates a test strip vial of an illustrative embodiment of the present invention including an information chip to provide read-write capability.

### Detailed Description of the Invention

According to an illustrative embodiment of the invention, an improved test strip vial for storing and dispensing test strips for use with a glucose meter is provided. The test strip vial of the illustrative embodiment is easy to use and facilitates handling of test strips for measuring glucose levels in a sample of blood. The test strip vial dispenses strips one-by-one and allows for the use of shorter test strips, thereby reducing costs and waste and increasing efficiency in the production of test strips. The use of the illustrative test strip vial according to the teachings of the present invention significantly reduces contamination, because the user is not required to manually handle a test strip to perform an analysis. The test strip vial further reduces waste by ensuring that a large number of test strips are efficiently stored and that the test strips are properly dispensed and sealed.

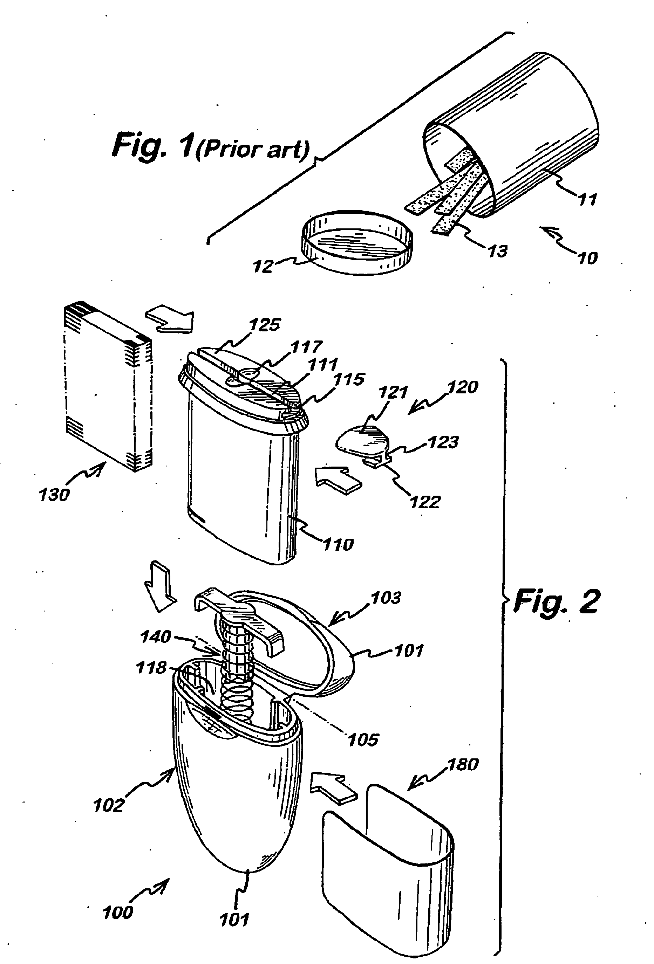

Figure 1 illustrates a conventional test strip vial 10 used for storing test strips for measuring glucose levels. As shown, the conventional test strip vial 10 comprises a cylindrical body 11 and a detached lid 12 for closing the vial. A supply of test strips 13 are stored loosely within the body 11. Removing and handling a single test strip from the conventional test strip vial 10 is generally a complicated task, particularly for diabetics. The conventional test strip vial 10 also does not efficiently utilize space, and stores a relatively small number of test strips.

Figure 2 is an exploded view of the test strip vial 100 of the illustrative embodiment of the present invention. The test strip vial 100 includes a number of components designed to facilitate one-by-one dispensing of test strips from the vial 100. As shown, the test strip vial 100 comprises a cassette 110 configured to received a stack of test strips 130 therein, a hermetically sealed outer shell or housing 101 defining an interior cavity 118 for holding the cassette 110 and biasing means 140 placed in the inner cavity 118 below the cassette 110 for pushing a stack of test strips stored in the cassette towards the top of the cassette 110. A slider 120 or other suitable dispensing mechanism is provided for dispensing test strips from the vial 100. A top surface 125 of the cassette 110 includes a longitudinal slot 111, which extends across the top surface 125 to allow the slider 120 to slide across the top of the cassette and dispense a test strip. As illustrated, the cassette 110 further includes a window 117 formed in the top surface 125 and intersecting the slot 111 to allow viewing of test strips stored in the cassette 110. When the strips are numbered, the user can determine the number of test strips remaining in the vial 100. In this way, a user is not confronted with the situation in which they have no access to a new vial and insufficient strips are left in the current vial. The user may decide, when insufficient number of strips remain in the current vial, to keep a new vial with them. A visual reading of the number on the strip may be replaced by an electronic reading of data storage means on the vial by a testing device. For example, the data storage means may be an EEPROM on the outside of the vial.

The test strip vial 100 may further include a sticker or a label 180 for displaying information related to the test strips stored therein. For example, the label 180 may display commercial information identifying the source of the test strips and/or coded information in the form of a bar code. The bar code may contain information identifying properties of the test strips, such as the batch code for the test strips, the date of expiration of the test strips, a calibration code, the initial number of test strips stored in a fully loaded vial, and so on.

The outer shell 101 of the test strip vial 100 comprises a base container 102 for holding the cassette 110 containing a supply of tests strips and a partially detachable vial lid 103 for closing and sealing the base container 102. According to the illustrative embodiment, the vial lid 103 is partially attached to the base container 102 by a hinge 105, to facilitate access to the supply of test strips stored in the cassette 110 without having to remove the lid entirely. The base container 102 includes a rim 109 formed at an upper edge of the base container. The rim 109 is configured to receive and hold a top portion of the cassette when the cassette 110 is inserted in the base container. The outer shell 101 is formed of a material that is durable, moisture resistant, easy to hold and robust, such as mouldable plastic. One skilled in the art will recognise that any suitable material for forming the housing 101 may be utilised.

As shown, the stack of test strips 130 comprises about fifty test strips in vertical alignment. However, the test strip vial 100 of the present invention is not limited to a stack of fifty test strips and may include any suitable number of stacked test strips. The illustrative vial 100 is designed to accommodate test strips having a smaller size than conventional test strips. According to the illustrative embodiment, the test strips 130 are between about 0.5 and about 2 centimetres in length. Preferably, the vial 100 stores and dispenses test strips that are less than about 1 centimetre in length, i.e., shorter than current test strip designs.

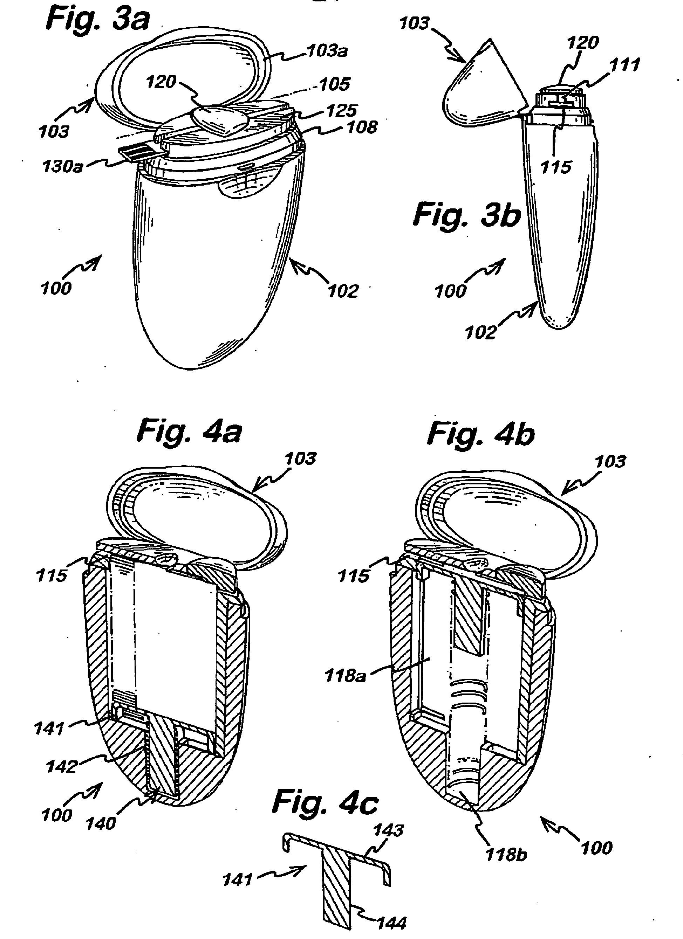

Figures 3a and 3b illustrate the assembled test strip vial 100 when the vial lid 103 is in an open position. Figure 3a is a perspective view of the test strip vial 100 and illustrates the dispensing of a foremost test strip 130a in the stack of test strips 130 from the vial 100. Figure 3b is a side view of the test strip vial 100 in the open position. According to the illustrative embodiment, the cassette 110 is inserted in the base container 102 and stores the stack of test strips 130 therein. When the cassette 110 is inserted in the base container 102, the top portion of the cassette sits on the rim 109 of the base container 102 and protrudes from the base container 102. The top portion of the cassette includes a hermetic sealing element 108 for sealing the vial 100 surrounding the perimeter of the cassette 110 where the cassette protrudes from the base container 102. The vial lid 103 includes a rim 103a corresponding to the hermetic seal 108. When the vial lid 103 is closed, the hermetic sealing element 108 and the rim 103a cooperate to seal the vial 100 and protect the test strips stored in the vial 100 from humidity and other harmful effects, which can damage the test strips and compromise test results. The hermetic seal 108 further ensures that when a test strip is dispensed from the vial 100, moisture is prevented from entering the base container cavity 118 and damaging the remaining test strips in the stack 130.

The hermetic seal 108 can also be positioned on the vial lid 103 or the base container 102 where it will meet the other part and snap shut to accommodate the pressure by its flexible characteristics. This also results in protection of the unused test strips stored in the vial 100 against humidity.

According to one practice, the material forming the outer shell 101 and/or the cassette 110 has desiccant properties, and/or desiccants are disposed in the interior space of the outer shell 101 and/or the cassette 110. Any humidity that may migrate into the test strip vial 100 is by these materials absorbed and neutralised, thereby further protecting the test strips from damage.

As shown, the top portion of the cassette 110, which projects from the base container 102 when the cassette is inserted in the base container, further includes a channel 115 formed therein for holding a foremost test strip 130a in the stack of test strips and guiding the foremost test strip 130a out of the vial 100. The channel 115 is sized and configured to hold only one test strip (i.e. the foremost test strip in the stack 130) at a time. As shown in Figure 3a, when the lid 103 is open, the vial exposes the foremost test strip 130a. The slider 120 is disposed in the slot 111 formed in the top surface 125 of the cassette 110. The slot 111 formed in the cassette 110 is in communication with the channel 115. A user dispenses the foremost test strip 130a by moving the slider 120 through the slot 111, thereby pushing the foremost test strip 130a through the channel 115 and out of the vial 100.

Referring back to Figure 2, the manual slider 120 is preferably formed of a moulded plastic material and comprises a button portion 121 that is sized and dimensioned to be pushed by a user, a runner 122 for pushing the test strip, and a connector 122 comprising for connecting the button 121 and the runner 122. As illustrated, the runner 122 is sized and dimensioned to fit in and slide through the channel 115, the connector 122 is sized to fit in and slide through the slot 111 and the button 121 slides across the top surface of the cassette 110. The three elements cooperate to push a test strip loaded in the channel 115 at least partially out of the channel, as shown in Figure 3a. According to one embodiment, the slider 120 may be fixed to biasing means, which may comprise a spring to bias the slider to a rest position. In this manner, the slider 120 automatically returns to the rest position after dispensing a test strip to allow loading of the next test strip in the stack into the channel 115 and subsequent dispensing of the next test strip.

Figures 4a and 4b are cross-sectional views along the length of the vial 100, illustrating the operation of the biasing means 140. Figure 4a illustrates a fully loaded vial 100 storing a stack 130 of about fifty test strips. In Figure 4b, the vial is nearly empty, with one remaining test strip in the stack 130. The biasing means 140 is disposed in the base container 102 and biases the test strip stack 130 towards the vial lid 103, such that the foremost test strip in the stack is always positioned in the channel 115. When the foremost test strip is removed from the stack 130, the remaining test strips in the stack advance by one. The biasing means includes a strip retainer 141 pressing against the last strip in the stack 130 and an elastic biasing element, illustrated as a spring 142. According to the illustrative embodiment, the spring comprises a constant force clock spring. When the vial 100 is assembled, the spring 142 is compressed by the stack of test strips 130 and applies a constant pressure to the stack 130. After the foremost strip 130a is removed from the stack, the biasing means 140 advances the stack towards the top of the cassette by one strip and loads the next strip in the stack in the channel 115. The next strip may be then dispensed for a subsequent analysis by activating the slider 120.

As shown in Figure 4b, the interior cavity 118 of the outer housing 101 includes a first hollow space 118a sized and dimension for holding the cassette and a second hollow space 118b below the first hollow space 118a sized and dimensioned for holding the spring 142. When the cassette is fully loaded with test strips, the spring 142 is fully compressed and the spring 142 and the base 144 of the retainer are positioned in the second hollow space 118b. As the test strips are dispensed, the restoring force of the spring causes the spring to expand and push the retainer 141 through the first hollow space 118a and towards the vial lid 103, while the base of spring is retained in the second hollow space 118b. According to the illustrative embodiment, the spring has a compressed length of about 20 mm, and a free length of about 60 mm, though one skilled in the art will recognise that the length and spring constant of the spring may be varied. A clock spring may be used as the biasing means to move between a full stack and an empty stack. Such a clock spring is the longest for the shortest "dead volume space". Also, such a clock spring has a more consistent force in the upward direction between the fresh and empty stack position.

As shown in Figure 4c, the retainer 141 of the illustrative embodiment is a T-shaped plastic element comprising a platform 143 for pressing against the test strips. The platform 143 has a substantially similar size and shape as a test strip, in order to apply a constant pressure across the last test strip in the stack 130. The retainer 141 further includes a base 144 connected to the platform for attaching the spring 142 to the retainer. One skilled in the art will recognise that the retainer 141 and biasing element 142 are not limited to the illustrative configurations and that any suitable devices for biasing the stack of test strips may be utilised.

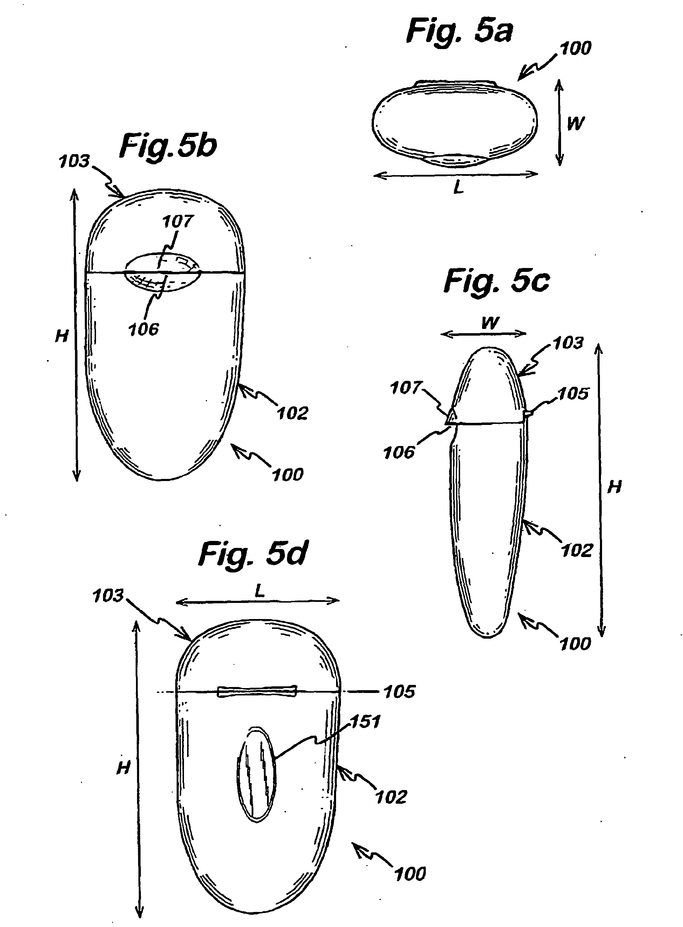

Figures 5a - 5d illustrate the details of the outer housing 101. The outer housing 101 is ergonomically shaped to facilitate holding of the vial 100 by a user. Figure 5a is a top view of the housing, illustrating the oval-shaped cross-section through the length (L) of the vial 100. Figure 5b is a frontal view of the vial 100, illustrating the shape of the vial through the height (H) of the vial 100. Figure 5c is a side view of the vial 100, illustrating the shape of the vial through the width (W) of the vial. The oval-shaped cross-section along the length, width and height of the vial 100 and the slightly tapered bottom allow a user to easily hold the vial 100 in the palm of the hand. As shown in Figure 5d, the illustrative outer housing 101 further includes a depression 151 on the back surface to facilitate handling by the user.

As shown in Figures 5b and 5c, the illustrative base container 102 includes a recessed portion 106 formed in the front side of the upper edge 109 of the base container. The lid 103 includes a protruding lip 107 formed in the lower edge of the lid corresponding to the recessed portion 106 in the partially attached base container 102. The recessed portion 106 and the lip 107 allow a user to open the vial 100 by pushing upwards on the lip 107, which swings the lid 103 about the hinge 105 and into an open position, as shown in Figure 2b.

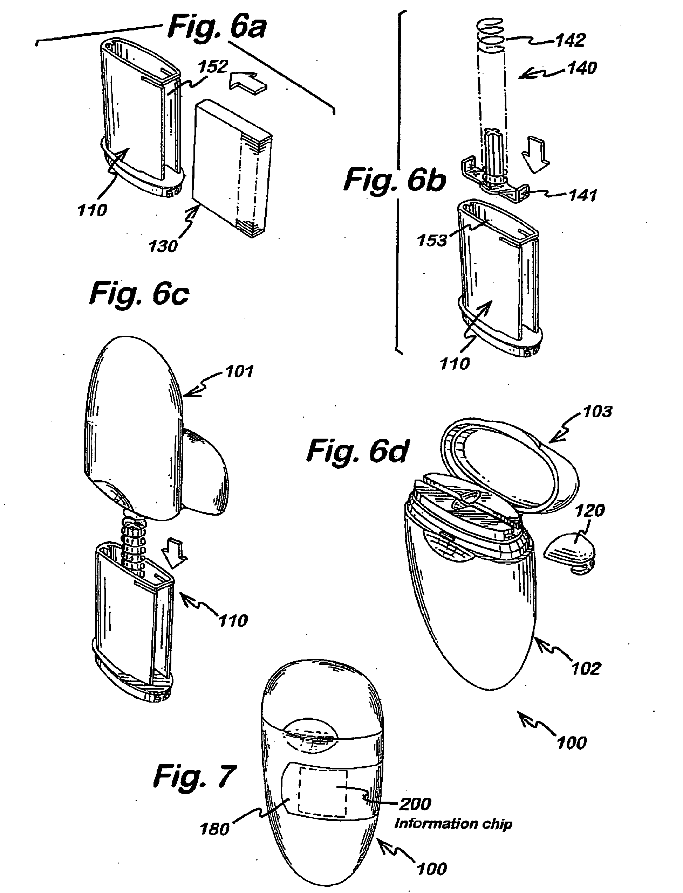

Figures 6a - 6d illustrate the steps involved in assembling the test strip vial 100 according to the teachings of the present invention. Figure 6a illustrates the step of loading a stack of test strips 130 into a cassette 110. The cassette 110 of the illustrative embodiment of the present invention preferably includes a first opening 152 formed in the side of the cassette to accommodate the stack 130. The stack of test strips 130 are pushed and clipped into the cassette via the opening 152. Next, as shown in Figure 6b, the biasing means 140 is clipped into the cassette 110 via a second opening 153 that is formed in the bottom of the cassette 110. The second opening 153 is sized and dimensioned to accommodate the retainer 141 of the biasing means 140. The biasing means 140 is inserted into the loaded cassette, such that the retainer 141 abuts the last strip in the stack 130. Then, as illustrates in Figure 6c, the outer housing 101 snaps onto the cassette 110, inserting the biasing means 140 and the cassette 110 into the cavity 118 of the outer housing 101. The step of inserting the cassette 110 into the cavity of the outer housing 101 compresses the spring 142. The compressed spring 142 imparts a constant pressure on the stack of test strips 130 and biases the stack towards the channel 115. Finally, as shown in Figure 6d, the slider 120 is clipped into the cassette 110, such that the runner 122 is positioned behind the foremost test strip in the channel 115, in position to dispense the foremost test strip.

The illustrative test strip vial 100 dispenses test strips for use with conventional glucose meters to measure glucose levels in a sample of blood. When it is time to measure a glucose level, the user slides the slider 120 through the channel 115 to push a foremost test strip 130a at least partially from the vial 100 to expose the electrodes of the test strip 130a, as illustrated in Figure 3a. The user aligns the exposed test strip 130a with a contact port of the glucose meter by holding the vial 100 in one hand and the glucose meter in the other hand. The user inserts the electrodes of the test strip 130a into the glucose meter and removes the vial 100. The friction and/or pressure of-contacts in the glucose meter-hold the strip 130a in place as the vial 100 is removed. The strip is now in position in the glucose meter, ready to accept a sample. The user lances skin to produce a small sample drop and transfer it to an application area on the strip 130a. The glucose meter then performs an electrochemical analysis of the sample to determine the amount of glucose in the sample and displays the glucose reading on a screen. The user then removes and discards the test strip 130a.

Of course, it will be understood that the user can also fully dispense the foremost test strip 130a from the vial 100 by sliding the slider 120 completely through the slot 111 and manually handle the test strip to perform the analysis.

Conventional glucose meters do not include a strip ejection button to eject the test strips from the meter after the analysis. Once the test strips are placed into the contact port of the meter, a user is required to manually remove the test strips from the contact port. However, removal by hand leads to possible contamination of the user by the blood on the strip, and vice versa, particularly when using the short strips that are enabled by the test strip vial 100 of the illustrative embodiment of the present invention. The test strip vial 100 of the present invention may be utilised with an adapter to facilitate testing using the test strips stored in the test strip vial 100 of the illustrative embodiment of the present invention. The adapter may include contacts for strip insertion and an ejection button enabling easy ejection of the used test strip.

Briefly, the adapter can be a clip-on adapter device for a standard glucose meter to convert the meter into, an integrated, single-unit testing device. The adapter comprises a lancing device, a test strip port for holding a test strip in close proximity to a puncture site, a strip connector for providing an electrical connection between the test strip and the electronics in the glucose meter and a connector for connecting the adapter to the glucose meter. The adapter includes a cocking mechanism for cocking the lancing device, a depth control mechanism for varying the penetration depth of the lancet and a trigger for actuating the lancet. The adapter may further include an adapter cap, including a pressure ring for facilitating the expression of blood from a juncture site.

Referring to Figure 7, the test strip vial 100 includes data storage means. According to the illustrative embodiment, the data storage means is an information chip 200, which has EPROM memory to provide read-write capability to the vial 100. According to the illustrative embodiment, the information chip 200 is incorporated in the label 180, though one skilled in the art will recognise that the information chip may be attached directly to the housing 101 in any suitable location. The information chip 200 electronically stores information related to the test strips, such as the expiration date, batch code, calibration information, lot number, number of strips, and other related information. According to one embodiment, the information chip 200 may also store glucose values obtained by an associated glucose meter that measures glucose levels using the test strips stored in the vial 100. The ability to store glucose values on the test strip vial 100 provides an extra copy of the glucose readings, in addition to the copy of the glucose readings that may be stored in the glucose meter. The redundant storage of the glucose values allows a user to send only the vial 100 back to the manufacturer for field claim support in the event of erroneous results, rather than having to send the entire glucose meter back.

According to the illustrative embodiment, the information chip 200 further includes built-in programmed software containing instructions for the glucose meter regarding the appropriate operating parameters for the associated test strips. The built-in programmed software can be transferred and read by the glucose meter in order to update or upgrade the operating parameters of the meter to reflect any changes in the test strips in the vial 100. For example, the software may instruct the glucose meter to adjust the time taken for a measurement, the portion of the measurement curve to be integrated, the potential to be poised by the potentiostat and other changes to accommodate the requirements and parameters of the test strips stored in the vial 100. In this manner, the information chip software allows new types of test strips to be used by existing glucose meters and automatically updates the existing glucose meters to accommodate the new test strips.

Furthermore, the information chip 200 can store calibration information related to the test strips to be read by the glucose meter to properly calibrate the glucose meter.

Additionally, the information chip 200 stores advertising information about the current strips, and displays this information on the associated glucose meter. The information chip 200 may further include information related to promotional offers, new releases of different strips and so on. For example, a retailer may provide diabetes products, information and advertisements to the diabetic patient via the test strip vial 100 by using the information chip 200.

The information chip 200 can further store personal information of the user to effectively personalise the glucose meter. For example, the information chip 200 may be programmed to display the user's name, address, emergency contact information, medical history and other personal information.

The information chip 200 is read during the alignment of the test strip 130a and vial 100 with the glucose meter. During this alignment there is an electrical contact between the meter and the information chip 200 on the vial 100. Means on both the glucose meter and the vial 100 will assure an easy and reliable contact between both.

The test strip vial 100 of the illustrative embodiment of the present invention provide significant advantages over current test strip vial designs. The illustrative test strip vial provides efficient storage and dispensing of test strips for measuring glucose levels while reducing contamination, degradation of the strips, cost and waste. The use of an information chip to convert the test strip vial into a "smart vial" provides redundant storage space for storing glucose values. The information chip further allows new test strips to be used with older model glucose meters and personalisation of the vial for the particular user.

It will of course be understood that the present invention has been described above purely by way of example, and modifications of detail can be made within the scope of the appended claims.

## Claims

1. A cassette (110) for holding and dispensing test strips (130) for analysing a sample of bodily fluid, the cassette (110) having a dispensing opening and being configured to hold a supply of test strips (130), the cassette (110) further including:
biasing means (140) for biasing test strips (130) held in the cassette (110) towards the dispensing opening;
retaining means for holding the test strips (130) in the cassette (110) against the bias of the biasing means (140); and
means for moving test strips (130), one at a time, out of the cassette (110) through the dispensing opening,
wherein the retaining means is an enclosing surface (125) at the end of the cassette (110) having the dispensing opening therein, and wherein the retaining means includes a longitudinal slot (111) extending along an outer side of the enclosing surface (125).

2. The cassette (110) according to claim 1, wherein the moving means is a slider (120).

3. A cassette (110) according to claim 1, wherein the biasing means (140) is attached to the cassette (110).

4. A cassette (110) according to claim 1 or claim 2, wherein the biasing means (140) is a helical spring.

5. A cassette (110) according to any one of the preceding claims, wherein the biasing means (140) is connected to a strip retainer for contacting the supply of test strips (130).

6. A cassette (110) according to any one of the preceding claims, wherein the enclosing surface (125) includes a channel in communication with the dispensing opening and configured to hold a single test strip from the supply of test strips (130).

7. A cassette (110) according to claim 6 when dependant on claim 2, wherein the longitudinal slot (111) holds the slider, such that the slider is in communication with the channel and movement of the slider towards the dispensing opening dispenses a single test strip held in the channel through the dispensing opening.

8. A cassette (110) according to claim 7, wherein the slider is biased to return the slider to its starting position after dispensing a test strip through the dispensing opening.

9. A cassette (110) according to any one of claims 5 to 8, further comprising a window formed in the enclosing surface (125) to allow a user to view a number on a surface of a test strip.

10. A cassette (110) according to any one of the preceding claims, further comprising desiccant material disposed in the cassette (110) for preserving the test strips (130) from humidity.

11. A cassette (110) according to any one of the preceding claims, wherein the cassette (110) is formed of desiccant material.

12. A cassette (110) according to any one of the preceding claims, wherein the test strips (130) have a length of one centimetre or less.

13. A cassette (110) according to any one of the preceding claims, wherein the cassette (110) is disposable.

14. A cassette (110) according to any one of the preceding claims, wherein the bodily fluid is blood and the test strips (130) are used for measurement of a glucose level in the blood.

15. A vial (100) comprising a cassette (110) according to any one of the preceding claims and an outer housing containing the cassette (110).

16. A vial (100) according to claim 1 wherein the biasing means (140) is attached to the outer housing and communicates with test strips (130) in the cassette (110) through a slot in the cassette (110).

17. A vial (100) according to claim 15 or claim 16, wherein the outer housing is ' formed of desiccant material.

18. A vial (100) according to any one of claims 15 to 17 wherein the outer housing has an oval-shaped cross-section.

19. A vial (100) according to any one of claims 15 to 18, wherein the outer housing includes a depression to facilitate holding of the vial (100) by a user.

20. A vial (100) according to any one of claims 15 to 19, wherein the cassette (110) includes a protruding portion that protrudes from the outer housing when the cassette (110) is inserted into the outer housing and a hermetic seal is disposed on the protruding portion.

21. A vial (100) according to any one of claims 15 to 20, wherein the outer housing comprises a lid.

22. A vial (100) according to claim 21, wherein the outer housing includes a recessed portion adjacent to an upper rim and the lid includes a protruding portion corresponding to the recessed portion to facilitate opening or removal of the lid.

23. A vial (100) according to any one of claims 15 to 22, further comprising data storage means carried by the vial (100) for storing information.

24. A vial (100) according to claim 23, wherein the data storage means is an electronic chip.

25. A vial (100) according to claim 24, wherein the electronic chip is an EPROM or an EEPROM.

26. A vial (100) according to any one of claims 23 to 25, wherein the information stored by the data storage means relates to information about a supply of test strips (130) stored in the vial (100).

27. A vial (100) according to claim 26, wherein the information comprises at least one of a calibration code for the supply of test strips (130), an expiration date of the supply of test strips (130), the batch code of the supply of test strips (130), the lot number of the supply of test strips (130) and the number of strips in the supply of test strips (130).

28. A vial (100) according to claim any one of claims 23 to 27, wherein the data storage means is adapted to store analyte values obtained by a testing device.

29. A vial (100) according to any one of claims 23 to 28, wherein the data storage means contains software which can be transferred to a testing device.

30. A vial (100) according to claim 29, wherein the software includes instructions for operating the testing device to accommodate a set of operating parameters for a supply of test strips (130) stored in the vial (100).

31. A vial (100) according to any one of claims 15 to 30, further comprising an adapter configured to connect to the contacts of a testing device.

32. A vial (100) according to any one of claims 15 to 31 for storing and dispensing test strips (130) for analysing a sample of bodily fluid comprising data storage means affixed to the housing for storing information related to the test strips (130).

33. A vial (100) according to claim 32, wherein the data storage means is an electronic chip.

34. A vial (100) according to claim 33, wherein the electronic chip is an EPROM or an EEPROM.

35. A vial (100) according to any one of claims 32 to 34, wherein the data storage means is adapted to store bodily fluid readings obtained by a testing device that performs an analysis using the test strips (130) stored in the vial (100).

36. A vial (100) according to any one of claims 32 to 35, wherein the data storage means-stores instructions for modifying the operating parameters of the testing device according to the type of test strips (130) stored in the vial (100).

37. A vial (100) according to any one of claims 32 to 36, wherein the data storage means-stores advertising information about the strips for display on a testing device.

38. A vial (100) according to any one of claims 32 to 37, wherein the data storage means stores personal information for a user of the vial (100).

39. A method of dispensing a test strip for measuring an analyte level in a sample of bodily fluid comprising:
dispensing a test strip from a cassette (110) as defined in any one of claims 1 to 14,
wherein after dispensing a test strip, the biasing means (140) applied to a supply of test strips (130) in the cassette (110) causes a further test strip to be made available for dispensing.

40. A method according to claim 39, wherein the cassette (110) is inserted into an outer housing before dispensing a test strip.

41. A method according to claim 39 or claim 40, further comprising the step of inserting the test strip into a testing device.

42. A method according to any one of claims 39 to 41, wherein the test strip is used to measure a glucose level in blood and the testing device is a glucose meter.

43. A method according to any one of claims 39 to 42, further comprising the step of disposing of the cassette (110) when the supply of test strips (130) is exhausted.

## Patentansprüche

1. Kassette (110) zum Halten und Ausgeben von Teststreifen (130) zum Analysieren einer Probe eines Körperfluides, wobei die Kassette (110) eine Ausgabeöffnung hat und so gestaltet ist, dass sie einen Vorrat an Teststreifen (130) hält, wobei die Kassette (110) weiter umfasst:
eine vorbelastende Einrichtung (140), um Teststreifen (130), die in der Kassette (110) gehalten werden, auf die Ausgabeöffnung zu vorzubelasten;
eine Rückhalteeinrichtung zum Halten der Teststreifen (130) in der Kassette (110) gegen die Vorbelastung der vorbelastenden Einrichtung (140); und
eine Einrichtung zum Bewegen von Teststreifen (130), jeweils einen zur Zeit, aus der Kassette (110) durch die Ausgabeöffnung,
wobei die Rückhalteeinrichtung eine einschließende Fläche (125) an dem Ende der Kassette (110) mit der Ausgabeöffnung darin ist und bei der die Rückhalteeinrichtung einen längs verlaufenden Schacht (111) umfasst, der sich entlang einer Außenseite der einschließenden Fläche (125) erstreckt.

2. Kassette (110) nach Anspruch 1, bei der die Bewegungseinrichtung ein Schieber (120) ist.

3. Kassette (110) nach Anspruch 1, bei der die vorbelastende Einrichtung (140) an der Kassette (110) befestigt ist.

4. Kassette (110) nach Anspruch 1 oder Anspruch 2, bei der die vorbelastende Einrichtung (140) eine Schraubenfeder ist.

5. Kassette (110) nach einem der vorangehenden Ansprüche, bei der die vorbelastende Einrichtung (140) mit einem Streifenhalter zum Berühren des Vorrats an Teststreifen (130) verbunden ist.

6. Kassette (110) nach einem der vorangehende Ansprüche, bei der die einschließende Fläche (125) einen Kanal in Verbindung mit der Ausgabeöffnung umfasst und so ausgestaltet ist, dass sie einen einzelnen Teststreifen aus dem Vorrat der Teststreifen (130) hält.

7. Kassette (110) nach Anspruch 6, soweit von Anspruch 2 abhängig, bei der der längs verlaufende Schacht (111) den Schieber hält, so dass der Schieber in Verbindung mit dem Kanal ist und die Bewegung des Schiebers auf die Ausgabeöffnung zu einen einzigen Teststreifen, der in dem Kanal gehalten wird, durch die Ausgabeöffnung ausgibt.

8. Kassette (110) nach Anspruch 7, bei der der Schieber vorbelastet ist, um den Schieber in seine Startposition zurückzuführen, nachdem ein Teststreifen durch die Ausgabeöffnung ausgegeben worden ist.

9. Kassette (110) nach einem der Ansprüche 5 bis 8, weiter mit einem Fenster, das in der einschließenden Fläche (125) gebildet ist, um es einem Benutzer zu erlauben, eine Zahl auf einer Oberfläche eines Teststreifens zu betrachten.

10. Kassette (110) nach einem der vorangehenden Ansprüche, weiter mit Trocknungsmaterial, das in der Kassette (110) zum Bewahren der Teststreifen (130) vor Feuchtigkeit angeordnet ist.

11. Kassette (110) nach einem der vorangehenden Ansprüche, bei der die Kassette (110) aus Trocknungsmaterial gebildet ist.

12. Kassette (110) nach einem der vorangehenden Ansprüche, bei der die Teststreifen (130) eine Länge von einem Zentimeter oder weniger haben.

13. Kassette (110) nach einem der vorangehenden Ansprüche, wobei die Kassette (110) entsorgbar ist.

14. Kassette (110) nach einem der vorangehenden Ansprüche, bei der das Körperfluid Blut ist und die Teststreifen (130) für das Messen eines Glukosewertes in dem Blut verwendet werden.

15. Phiole (100), die eine Kassette (110) nach einem der vorangehenden Ansprüche und ein Außengehäuse, dass die Kassette (110) enthält, aufweist.

16. Phiole (100) nach Anspruch 15, bei der die vorbelastende Einrichtung (140) an dem Außengehäuse befestigt ist und mit den Teststreifen (130) in der Kassette (110) durch einen Schacht in der Kassette (110) in Verbindung ist.

17. Phiole (100) nach Anspruch 15, oder Anspruch 16, bei der das Außengehäuse aus einem Trocknungsmaterial gebildet ist.

18. Phiole (100) nach einem der Ansprüche 15 bis 17, bei der das Außengehäuse eine ovalförmigen Querschnitt hat.

19. Phiole (100) nach einem der Ansprüche 15 bis 18, bei der das Außengehäuse eine Einsenkung umfasst, um das Halten der Phiole (100) durch einen Benutzer zu vereinfachen.

20. Phiole (100) nach einem der Ansprüche 15 bis 19, bei der die Kassette (110) einen hervorstehenden Bereich umfasst, der von dem Außengehäuse hervorsteht, wenn die Kassette (110) in das Außengehäuse eingeführt ist, und eine hermetische Dichtung um den hervorstehenden Bereich angeordnet ist.

21. Phiole (100) nach einem der Ansprüche 15 bis 20, bei der das Außengehäuse einen Deckel aufweist.

22. Phiole (100) nach Anspruch 21, bei der das Außengehäuse einen ausgenommenen Bereich benachbart einem oberen Rand umfasst und der Deckel einen hervorstehenden Bereich umfasst, der dem ausgenommenen Bereich entspricht, um das Öffnen oder Entfernen des Deckels zu vereinfachen.

23. Phiole (100) nach einem der Ansprüche 15 bis 22, weiter mit einem Datenspeichereinrichtung, die von der Phiole (100) gehalten wird, zum Speichern von Information.

24. Phiole (100) nach Anspruch 23, bei der die Datenspeichereinrichtung ein elektronischer Chip ist.

25. Phiole (100) nach Anspruch 24, bei der der elektronische Chip ein EPROM oder ein EEPROM ist.

26. Phiole (100) nach einem der Ansprüche 23 bis 25, bei der die Information, die von der Datenspeichereinrichtung gespeichert wird, sich auf Information über einen Vorrat an Teststreifen (130) bezieht, der in der Phiole (100) gelagert ist.

27. Phiole (100) nach Anspruch 26, bei der die Information wenigstens eines aus einem Kalibriercode für den Vorrat der Teststreifen (130), einem Ablaufdatum für den Vorrat an Teststreifen (130), den Batch-Code des Vorrats von Teststreifen (130), die Losnummer des Vorrats an Teststreifen (130) und die Anzahl von Streifen in dem Vorrat an Teststreifen (130) aufweist.

28. Phiole (100) nach einem der Ansprüche 23 bis 27, bei der die Datenspeichereinrichtung dazu ausgelegt ist, Analytenwerte zu speichern, die von einem Testgerät erhalten worden sind.

29. Phiole (100) nach einem der Ansprüche 23 bis 28, bei der die Datenspeichereinrichtung Software enthält, die an ein Testgerät übertragen werden kann.

30. Phiole (100) nach Anspruch 29, bei der die Software Befehle zum Betreiben des Testgerätes umfasst, um einen Satz von Betriebsparametern für einen Vorrat an Teststreifen (130), der in der Phiole (100) gelagert ist, bereitzustellen.

31. Phiole (100) nach einem der Ansprüche 15 bis 30, weiter mit einem Adapter, der so gestaltet ist, dass er an die Kontakte eines Testgerätes anschließt.

32. Phiole (100) nach einem der Ansprüche 15 bis 31 zum Lagern und Ausgeben von Teststreifen (130) zum Analysieren einer Probe von Körperflüssigkeit, mit einer Datenspeichereinrichtung, die an dem Gehäuse befestigt ist, zum Speichern von Information, die die Teststreifen (130) betrifft.

33. Phiole (100) nach Anspruch 32, bei der die Datenspeichereinrichtung ein elektronischer Chip ist.

34. Phiole (100) nach Anspruch 33, bei der der elektronische Chip ein EPROM oder ein EEPROM ist.

35. Phiole (100) nach einem der Ansprüche 32 bis 34, bei der die Datenspeichereinrichtung dazu ausgelegt ist, Ablesungen für Körperfluid zu speichern, die von einem Testgerät erhalten worden sind, welches eine Analyse durchführt, bei der die Teststreifen (130), die in der Phiole (100) gelagert sind, verwendet werden.

36. Phiole (100) nach einem der Ansprüche 32 bis 35, bei der die Datenspeichereinrichtungen Befehle zum Abändern der Betriebsparameter des Testgerätes entsprechend dem Typ der Teststreifen (110), die in der Phiole (100) gelagert werden, speichert.

37. Phiole (100) nach einem der Ansprüche 32 bis 36, bei der die Datenspeichereinrichtung Werbeinformation über die Streifen zur Anzeige auf einem Testgerät speichert.

38. Phiole (100) nach einem der Ansprüche 32 bis 37, bei der die Datenspeichereinrichtung persönliche Information für einen Benutzer der Phiole (100) speichert.

39. Verfahren zum Ausgeben eines Teststreifens zum Messen eines Analytenwerts in einer Probe von Körperfluid, das aufweist:
Ausgeben eines Teststreifens aus einer Kassette (110), wie sie in einem der Ansprüche 1 bis 14 definiert ist,
wobei nach dem Ausgeben eines Teststreifens die vorbelastende Einrichtung (140), die bei einem Vorrat von Teststreifen (130) in der Kassette (110) angelegt ist, bewirkt, dass ein weiterer Teststreifen für das Ausgeben verfügbar gemacht wird.

40. Verfahren nach Anspruch 39, bei dem die Kassette (110) in ein Außengehäuse eingesetzt wird, bevor ein Teststreifen ausgegeben wird.

41. Verfahren nach Anspruch 39 oder 40, weiter mit dem Schritt des Einsetzens des Teststreifens in ein Testgerät.

42. Verfahren nach einem der Ansprüche 39 bis 41, bei dem der Teststreifen verwendet wird, den Glukosewert in Blut zu messen, und das Testgerät ein Glukosemessgerät ist.

43. Verfahren nach einem der Ansprüche 39 bis 42, weiter mit dem Schritt des Entsorgens der Kassette (110), wenn der Vorrat an Teststreifen (130) erschöpft ist.

## Revendications

1. Cartouche (110) destinée à contenir et distribuer des bandes d'essai (130) pour l'analyse d'un échantillon de liquide biologique, la cartouche (110) comportant une ouverture de distribution et étant configurée pour contenir une réserve de bandes d'essai (130), la cartouche (110) comprenant en outre :
• des moyens de sollicitation (140) pour pousser les bandes d'essai (130) contenues dans la cartouche (110) vers l'ouverture de distribution ;
• des moyens de retenue pour maintenir les bandes d'essai (130) dans la cartouche (110) à l'encontre de la force des moyens de sollicitation (140) ; et
• des moyens de déplacement pour faire sortir les bandes d'essai (130), une à la fois, de la cartouche (110) à travers l'ouverture de distribution,
dans laquelle les moyens de retenue sont une surface d'enceinte (125) située à l'extrémité de la cartouche (110) et comportant l'ouverture de distribution dans celle-ci, et dans laquelle les moyens de retenue comprennent une fente longitudinale (111) s'étendant le long d'un côté extérieur de la surface d'enceinte (125).

2. Cartouche (110) selon la revendication 1, dans laquelle les moyens de déplacement sont un curseur (120).

3. Cartouche (110) selon la revendication 1, dans laquelle les moyens de sollicitation (140) sont fixés à la cartouche (110).

4. Cartouche (110) selon la revendication 1 ou la revendication 2, dans laquelle les moyens de sollicitation (140) sont un ressort hélicoïdal.

5. Cartouche (110) selon l'une quelconque des revendications précédentes, dans laquelle les moyens de sollicitation (140) sont reliés à un dispositif de retenue de bande destiné à être en contact avec la réserve de bandes d'essai (130).

6. Cartouche (110) selon l'une quelconque des revendications précédentes, dans laquelle la surface d'enceinte (125) comprend un canal communiquant avec l'ouverture de distribution et configuré pour contenir une seule bande d'essai issue de la réserve de bandes d'essai (130).

7. Cartouche (110) selon la revendication 6 liée à la revendication 2, dans laquelle la fente longitudinale (111) retient le curseur, de sorte que le curseur communique avec le canal et le déplacement du curseur vers l'ouverture de distribution provoque la distribution d'une seule bande d'essai contenue dans le canal à travers l'ouverture de distribution.

8. Cartouche (110) selon la revendication 7, dans laquelle le curseur est sollicité pour revenir à sa position de départ après avoir distribué une bande d'essai à travers l'ouverture de distribution.

9. Cartouche (110) selon l'une quelconque des revendications 5 à 8, comprenant en outre une fenêtre formée dans la surface d'enceinte (125) pour permettre à un utilisateur de voir un numéro sur une surface d'une bande d'essai.

10. Cartouche (110) selon l'une quelconque des revendications précédentes, comprenant en outre un matériau déshydratant disposé dans la cartouche (110) pour protéger les bandes d'essai (130) de l'humidité.

11. Cartouche (110) selon l'une quelconque des revendications précédentes, dans laquelle la cartouche (110) est formée d'un matériau déshydratant.

12. Cartouche (110) selon l'une quelconque des revendications précédentes, dans laquelle les bandes d'essai (130) ont une longueur maximale d'un centimètre.

13. Cartouche (110) selon l'une quelconque des revendications précédentes, dans laquelle la cartouche (110) est jetable.

14. Cartouche (110) selon l'une quelconque des revendications précédentes, dans laquelle le liquide biologique est du sang et les bandes d'essai (130) servent à mesurer un niveau de glucose dans le sang.

15. Fiole (100) comprenant une cartouche (110) selon l'une quelconque des revendications précédentes et un boîtier externe contenant la cartouche (110).

16. Fiole (100) selon la revendication 15, dans laquelle les moyens de sollicitation (140) sont fixés au boîtier externe et communiquent avec les bandes d'essai (130) de la cartouche (110) à travers une fente de la cartouche (110).

17. Fiole (100) selon la revendication 15 ou la revendication 16, dans laquelle le boîtier externe est formé d'un matériau déshydratant.

18. Fiole (100) selon l'une quelconque des revendications 15 à 17, dans laquelle le boîtier externe a une section transversale de forme ovale.

19. Fiole (100) selon l'une quelconque des revendications 15 à 18, dans laquelle le boîtier externe comprend un creux destiné à faciliter la préhension de la fiole (100) par un utilisateur.

20. Fiole (100) selon l'une quelconque des revendications 15 à 19, dans laquelle la cartouche (110) comprend une partie saillante qui fait saillie à partir du boîtier externe lorsque la cartouche (110) est insérée dans le boîtier externe et un joint hermétique est disposé sur la partie saillante.

21. Fiole (100) selon l'une quelconque des revendications 15 à 20, dans laquelle le boîtier externe comprend un couvercle.

22. Fiole (100) selon la revendication 21, dans laquelle le boîtier externe comprend une partie creuse adjacente à un bord supérieur et le couvercle comprend une partie saillante correspondant à la partie creuse pour faciliter l'ouverture ou le retrait du couvercle.

23. Fiole (100) selon l'une quelconque des revendications 15 à 22, comprenant en outre des moyens de stockage de données solidaires de la fiole (100) et destinés à stocker des informations.

24. Fiole (100) selon la revendication 23, dans laquelle les moyens de stockage de données sont une puce électronique.

25. Fiole (100) selon la revendication 24, dans laquelle la puce électronique est une mémoire morte programmable électriquement ou une mémoire morte programmable effaçable électriquement.

26. Fiole (100) selon l'une quelconque des revendications 23 à 25, dans laquelle les informations stockées par les moyens de stockage de données sont des informations relatives à une réserve de bandes d'essai (130) stockées dans la fiole (100).

27. Fiole (100) selon la revendication 26, dans laquelle les informations comprennent au moins une information parmi un code d'étalonnage de la réserve de bandes d'essai (130), une date d'expiration de la réserve de bandes d'essai (130), le code de lot de la réserve de bandes d'essai (130), le numéro de lot de la réserve de bandes d'essai (130) et le nombre de bandes de la réserve de bandes d'essai (130).

28. Fiole (100) selon l'une quelconque des revendications 23 à 27, dans laquelle les moyens de stockage de données sont aptes à stocker des valeurs d'analyte obtenues à partir d'un dispositif d'analyse.

29. Fiole (100) selon l'une quelconque des revendications 23 à 28, dans laquelle les moyens de stockage de données contiennent un logiciel qui peut être transféré vers un dispositif d'analyse.

30. Fiole (100) selon la revendication 29, dans laquelle le logiciel comprend des instructions destinées à faire fonctionner le dispositif d'analyse pour prendre en compte un jeu de paramètres d'exploitation d'une réserve de bandes d'essai (130) stockées dans la fiole (100).

31. Fiole (100) selon l'une quelconque des revendications 15 à 30, comprenant en outre un adaptateur configuré pour se connecter aux contacts d'un dispositif d'analyse.

32. Fiole (100) selon l'une quelconque des revendications 15 à 31, destinée à stocker et distribuer des bandes d'essai (130) pour l'analyse d'un échantillon de liquide biologique et comprenant des moyens de stockage de données fixés au boîtier pour stocker des informations relatives aux bandes d'essai (130).

33. Fiole (100) selon la revendication 32, dans laquelle les moyens de stockage de données sont une puce électronique.

34. Fiole (100) selon la revendication 33, dans laquelle la puce électronique est une mémoire morte programmable électriquement ou une mémoire morte programmable effaçable électriquement.

35. Fiole (100) selon l'une quelconque des revendications 32 à 34, dans laquelle les moyens de stockage de données sont aptes à stocker des lectures relatives à un liquide biologique obtenues par un dispositif d'analyse qui effectue une analyse au moyen des bandes d'essai (130) stockées dans la fiole (100).

36. Fiole (100) selon l'une quelconque des revendications 32 à 35, dans laquelle les moyens de stockage de données stockent des instructions permettant de modifier les paramètres d'exploitation du dispositif d'analyse en fonction du type de bandes d'essai (130) stockées dans la fiole (100).

37. Fiole (100) selon l'une quelconque des revendications 32 à 36, dans laquelle les moyens de stockage de données stockent des informations publicitaires relatives aux bandes pour affichage sur un dispositif d'analyse.

38. Fiole (100) selon l'une quelconque des revendications 32 à 37, dans laquelle les moyens de stockage de données stockent des informations personnelles destinées à l'utilisateur de la fiole (100).

39. Méthode permettant de distribuer une bande d'essai pour mesurer un niveau d'analyte dans un échantillon de liquide biologique, comprenant l'étape consistant à :
distribuer une bande d'essai à partir d'une cartouche (110) telle que définie dans l'une quelconque des revendications 1 à 14,
dans laquelle, après avoir distribué une bande d'essai, les moyens de sollicitation (140) appliqués à une réserve de bandes d'essai (130) dans la cartouche (110) provoquent la libération d'une autre bande d'essai pour distribution.

40. Méthode selon la revendication 39, dans laquelle la cartouche (110) est insérée dans un boîtier externe avant la distribution d'une bande d'essai.

41. Méthode selon la revendication 39 ou la revendication 40, comprenant en outre l'étape consistant à insérer la bande d'essai dans un dispositif d'analyse.

42. Méthode selon l'une quelconque des revendications 39 à 41, dans laquelle la bande d'essai sert à mesurer un niveau de glucose dans le sang et le dispositif d'analyse est un glucomètre.

43. Méthode selon l'une quelconque des revendications 39 à 42, comprenant en outre l'étape consistant à jeter la cartouche (110) lorsque la réserve de bandes d'essai (130) est épuisée.
